# EUROPEAN PATENT SPECIFICATION

(11) **EP 1 369 512 B1**
(45) Date of publication and mention of the grant of the patent: **07.03.2007**
(21) Application number: 02016234.3
(22) Date of filing: 19.07.2002
(51) Int. Cl.: D03D 15/00

(54) **Fabric suitable to provide a barrier effect against magnetic and electromagnetic fields and/or metallotherapy effects**
Gewebe zur Erzeugung einer Sperrwirkung gegen magnetische und elektromagnetische Felder und/oder metallotherapeutische Wirkungen
Tissu à donner un effet barrière aux champs magnétiques ou electromagnétiques et/ou des effets metallothérapeutiques

(30) Priority: 03.06.2002 IT MI20021200
(43) Date of publication of application: 10.12.2003
(73) Proprietor: Gafitex s.r.l., 46040 Guidizzolo (Mantova) (IT)
(72) Inventor: Botturi, Luca, 46040 Guidizzolo (IT)
(74) Representative: Modiano, Micaela Nadia

(56) References cited:
- WO-A-89/12706
- US-A- 5 906 004

## Description

The present invention relates to a fabric suitable to provide a barrier effect against magnetic and electromagnetic fields and/or metallotherapy effects. Such a fabric is known WO-A-89 12706.

The theory on which metallotherapy is based likens the human body to a voltaic pile, starting from the observation that if a plate of positive metal (for example copper) is applied to the spinal region and a plate of negative metal (for example zinc) is applied to the abdominal region and these two plates are connected to the two poles of a microammeter, a flow of current is observed between the two plates, i.e., through the human body.

Connecting the two plates with an electrically conducting wire produces a short-circuit in the human body and an ionization that causes the penetration of ions of the positive metal into the body of the subject.

This penetration can still occur, albeit more slowly and to a lesser extent, if the plates are mutually connected by silicon, which acts as a conductor.

The metallic oligoelements that penetrate the body act on the terminations of the sympathetic system, affected by a spasm, which can be ascribed to clumps (flocculates) of leukocytes that obstruct the capillaries, and are able to cause accordingly a relaxation of the spasm, eliminating the white cells that are dead or liquefied in the general circulation.

Essentially, according to this theory, the application of positive and negative metals to the surface of the skin produces an infinitesimal current that is capable of healing or improving certain deficiencies or certain functional or nervous chronic afflictions in which acidosis is the rule. The basic concept that the human body, its tissues, its humors are used as the liquid or compound of a dry cell and is converted, with the two metals (the positive one and the negative one) and the silicon that acts as their conductor, into a true voltaic pile.

It should be observed that the "human pile", whose living electrolyte is more or less acid, therefore operates actively or not depending on its individual acidity. Therefore, the resulting intracellular current is able to self-regulate, since its intensity depends on organic acidosis.

The effects that can be obtained by means of metallotherapy are:
general stimulation of the body;
stimulation of the nutritional function;
natural and spontaneous elimination of organic residues and flocculates;
improvement of blood circulation;
cessation of various pains;
recovery of nervous energy.

In particular, the use of the copper-zinc combination prevents the onset of rheumatic pain and reduces emotional stress states. The use of silicon eliminates excessive perspiration, yet keeps the skin elastic and soft without making it dry.

It is also known that some metals, such as for example copper, have a shielding effect on electromagnetic waves.

The widespread use of electric and/or electronic devices has brought to the forefront the problem of protecting people from the potentially harmful effects of electromagnetic waves. This problem is felt increasingly, in view of the enormous growth experienced in recent years by radios, computers, television sets and most of all wireless telephones.

The aim of the present invention is to provide a fabric, intended for the most disparate applications, that can perform effectively a shielding action against magnetic and electromagnetic fields and/or, if placed in direct contact with the human body, can also have metallotherapy effects.

Within this aim, an object of the present invention is to provide a fabric that can be produced with currently commercially available weaving machines.

Another object of the invention is to provide a fabric that can be manufactured at competitive costs.

This aim and these and other objects that will become better apparent hereinafter are achieved by a fabric suitable to produce a barrier effect against magnetic and electromagnetic fields and/or metallotherapy effects, characterized in that it has a weft-and-warp structure that is formed at least partially by means of threads that comprise copper, threads that comprise zinc, and threads that comprise silicon.

Further characteristics and advantages will become better apparent from the following detailed description of a preferred but not exclusive embodiment of the fabric according to the present invention, illustrated only by way of non-limitative example in the accompanying drawings, wherein:
Figure 1 is a view of the fabric according to the invention;
Figure 2 is an enlarged-scale view of a portion of Figure 1.

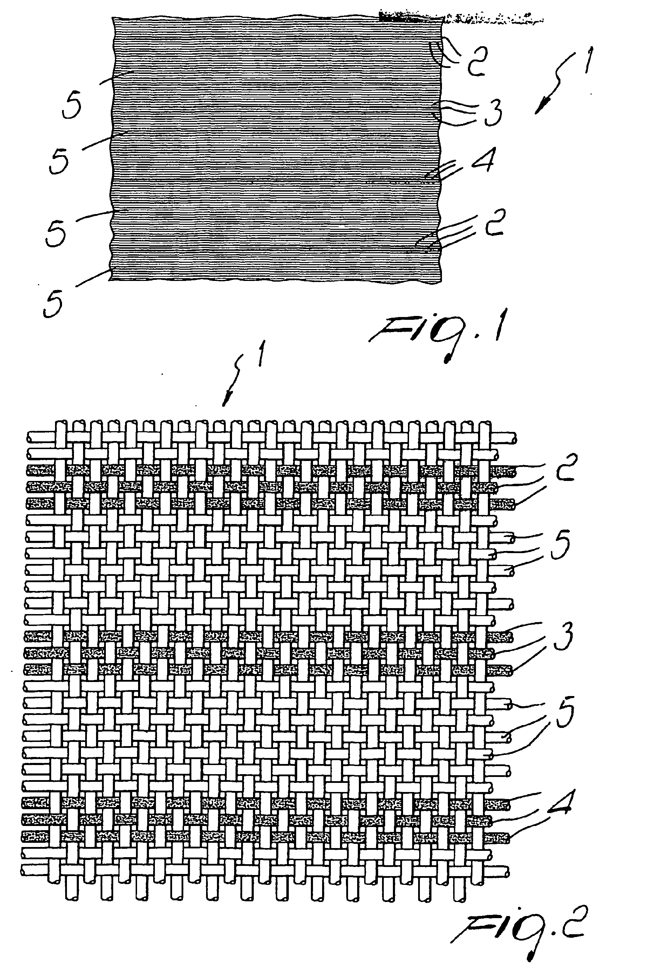

With reference to the figures, the fabric according to the invention, generally designated by the reference numeral 1, comprises a weft-and-warp structure, in which at least some of the warp and/or weft threads are constituted by threads 2 that comprise copper, threads 3 that comprise zinc, and threads 4 that comprise silicon.

The copper, zinc and silicon can be provided in filament form, so that they can be processed directly by the weaving machine to form the fabric, or can be embedded, in the form of powder or fibers or particles, in the natural or synthetic fibers of threads used to produce the fabric.

The threads 2, 3, 4 comprising copper, zinc and silicon might be used exclusively in the warp or exclusively in the weft or both in the weft and in the warp.

Depending on the requirements, the threads 2 comprising copper and/or the threads 3 comprising zinc and/or the threads 4 comprising silicon are alternated with weft or warp threads 5 made of synthetic or natural yarns.

Preferably, there are three sets of threads that are mutually alternated and repeated sequentially, according to a preset spacing, in the fabric, along the weft and/or the warp, and respectively: a first set of threads 2 comprising copper, a second set of threads 3 comprising zinc, and a third set of threads 4 comprising silicon. Threads 5 made of synthetic or natural yarns may be inserted between the threads of a same set. Moreover, the various sets of threads 2, 3 and 4 can be mutually joined or spaced by interposing sets of threads 5 made of synthetic or natural yarns.

The fabric according to the invention can be used in a wide range of fields.

Merely by way of example, the fabric according to the invention can be used in the production of items of clothing or components of items of clothing such as socks, stockings, underpants, undershirts, T-shirts, body suits, scarves, caps, balaclavas, gloves, earmuffs, overalls, pajamas, gussets for underwear, gussets for bathing costumes, collars, linings for clothing or linings in general, or in the manufacture of sanitary articles such as footlets, support belts, girdles, ankle bands, knee bands, shoulder bandages, gauzes, headbands, wrist bands, mouth masks, or in the production of footwear or components for footwear such as shoes, slippers, sandals, boots, insole, footwear linings, paddings for footwear, or in the production of interior decoration items such as curtains, drapes, carpets, upholstery, coverings of pieces of furniture such as chairs, sofas and armchairs, or in the production of bed linen such as sheets, pillow cases, mattress covers, and in the production of other articles.

If the fabric is used in the production of articles that do not make direct contact with the body of people, by way of the presence of the copper, zinc and silicon threads it performs a barrier action against magnetic and electromagnetic fields. The effectiveness of this barrier effect depends on the position of the fabric with respect to the position of the magnetic field and/or with respect to the source of emission of the electromagnetic waves and to the user. The user needs not be fully wrapped in the fabric, since experimental tests have shown that the barrier effect reaches as far as a few tens of centimeters from the fabric.

If the fabric according to the invention makes direct contact with the skin of the person who uses it, in addition to performing the mentioned barrier effect against magnetic and electromagnetic fields, the combined action of copper, zinc and silicon also has a metallotherapy effect, achieving beneficial effects at the local level as regards blood circulation, prevention or reduction of articular or rheumatic pains, regularization of sweating, and, at the systemic level, as regards the reduction of states of emotional stress.

In practice it has been observed that the fabric fully achieves the intended aim, since by way of the presence of copper, zinc and silicon it provides a barrier effect against magnetic fields and electromagnetic waves and, if placed in direct contact with the skin, also has metallotherapy effects.

Another advantage of the fabric according to the invention is that it causes no discomfort to the user.

The present invention is susceptible of modifications and variations, all of which are within the scope of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A fabric suitable to produce a barrier effect against magnetic and electromagnetic fields and/or metallotherapy effects, **characterized in that** it has a weft-and-warp structure that is formed at least partially by means of threads that comprise copper, threads that comprise zinc, and threads that comprise silicon.

2. The fabric according to claim 1, **characterized in that** said threads are warp threads.

3. The fabric according to claim 1, **characterized in that** said threads are weft threads.

4. The fabric according to one or more of the preceding claims, **characterized in that** said threads are present both in the weft and in the warp.

5. The fabric according to one or more of the preceding claims, **characterized in that** said threads comprising copper and/or said threads comprising zinc and/or said threads comprising silicon are alternated with weft or warp threads made of synthetic or natural yarns.

6. The fabric according to one or more of the preceding claims, **characterized in that** it comprises three sets of threads: a first set of threads comprising copper, a second set of threads comprising zinc, and a third set of threads comprising silicon, said three sets of threads being warp threads and being mutually alternated sequentially in the direction of the weft.

7. The fabric according to one or more of the preceding claims, **characterized in that** it comprises three sets of threads: a first set of threads comprising copper, a second set of threads comprising zinc, and a third set of threads comprising silicon, said three sets of threads being weft threads and being mutually alternated sequentially in the direction of the warp.

8. The fabric according to claim 6, **characterized in that** said three sets of threads are mutually spaced by interposing threads made of synthetic or natural yarns.

9. An item of clothing or a component of an item of clothing, **characterized in that** it is made of a fabric according to one or more of the preceding claims.

10. An item of sanitary clothing, particularly of the kind of a girdle, ankle, bandage, knee bandage, shoulder bandage, gauze, wrist band, **characterized in that** it is made of a fabric according to one or more of the preceding claims.

11. An item of footwear or footwear component, **characterized in that** it is made of a fabric according to one or more of the preceding claims.

12. An item for interior decoration, **characterized in that** it comprises a fabric according to one or more of the preceding claims.

13. A covering for interior decoration components, **characterized in that** it comprises a fabric according to one or more of the preceding claims.

14. A bed linen item, **characterized in that** it comprises a fabric according to one or more of the preceding claims.

## Patentansprüche

1. Ein Gewebe, das geeignet ist, eine Sperrwirkung gegen magnetische und elektromagnetische Felder und/oder metallotherapeutische Wirkungen zu erzeugen, **dadurch gekennzeichnet, dass** es eine Schuss-und-Ketten Struktur aufweist, die zumindest teilweise mittels Fäden die Kupfer umfassen, Fäden die Zink umfassen, und Fäden die Silizium umfassen, gebildet ist.

2. Das Gewebe gemäß Anspruch 1, **dadurch gekennzeichnet, dass** die Fäden Kettfäden sind.

3. Das Gewebe gemäß Anspruch 1, **dadurch gekennzeichnet, dass** die Fäden Schussfäden sind.

4. Das Gewebe gemäß einem oder mehreren der vorhergehenden Ansprüche, **dadurch gekennzeichnet, dass** die Fäden sowohl im Schuss, als auch in der Kette vorhanden sind.

5. Das Gewebe gemäß einem oder mehreren der vorhergehenden Ansprüche, **dadurch gekennzeichnet, dass** die Kupfer umfassenden Fäden, und/oder die Zink umfassenden Fäden, und/oder die Silizium umfassenden Fäden, sich mit aus synthetischen oder natürlichen Garnen hergestellten Schuss- und Kettfäden abwechseln.

6. Das Gewebe gemäß einem oder mehreren der vorhergehenden Ansprüche, **dadurch gekennzeichnet, dass** es drei Sätze Fäden umfasst: einen ersten Satz Fäden, Kupfer umfassend, einen zweiten Satz Fäden, Zink umfassend, und einen dritten Satz Fäden, Silizium umfassend, wobei die drei Sätze Kettfäden sind und sich in Richtung des Schusses nacheinander gegenseitig abwechseln.

7. Das Gewebe gemäß einem oder mehreren der vorhergehenden Ansprüche, **dadurch gekennzeichnet, dass** es drei Sätze von Fäden umfasst: einen ersten Satz Fäden, Kupfer umfassend, einen zweiten Satz Fäden, Zink umfassend, und einen dritten Satz Fäden, Silizium umfassend, wobei die drei Sätze Schussfäden sind und sich in Richtung der Kette nacheinander gegenseitig abwechseln.

8. Das Gewebe gemäß Anspruch 6, **dadurch gekennzeichnet, dass** die drei Sätze von Fäden gegenseitig mittels eingefügter Fäden, hergestellt aus synthetischen oder natürlichen Garnen, in Abständen angeordnet sind.

9. Ein Bekleidungsgegenstand oder eine Komponente eines Bekleidungsgegenstands, **dadurch gekennzeichnet, dass** er aus einem Gewebe gemäß einem oder mehreren der vorhergehenden Ansprüche hergestellt ist.

10. Ein Sanitärbekleidungsgegenstand, insbesondere in der Form eines Gurts, einer Gelenkbandage, Kniebandage, Schulterbandage, Gaze, einem Armband, **dadurch gekennzeichnet, dass** er aus einem Gewebe gemäß einem oder mehreren der vorhergehenden Ansprüche hergestellt ist.

11. Ein Schuhwerkgegenstand oder eine Schuhwerkkomponente, **dadurch gekennzeichnet, dass** er/sie aus einem Gewebe gemäß einem oder mehreren der vorhergehenden Ansprüche hergestellt ist.

12. Ein Gegenstand zur Innenausstattung, **dadurch gekennzeichnet, dass** er ein Gewebe gemäß einem oder mehreren der vorhergehenden Ansprüche umfasst.

13. Eine Abdeckung für Innenausstattungskomponenten, **dadurch gekennzeichnet, dass** sie ein Gewebe gemäß einem oder mehreren der vorhergehenden Ansprüche umfasst.

14. Ein Bettwäschegegenstand, **dadurch gekennzeichnet, dass** er ein Gewebe gemäß einem oder mehreren der vorhergehenden Ansprüche umfasst.

## Revendications

1. Tissu apte à produire un effet barrière contre les champs magnétiques et électromagnétiques et/ou des effets métallothérapeutiques, **caractérisé en ce qu'**il présente une structure à trame et à chaîne qui est formée au moins en partie au moyen de fils comprenant du cuivre, de fils comprenant du zinc, et de fils comprenant du silicium.

2. Tissu selon la revendication 1, **caractérisé en ce que** lesdits fils sont des fils de chaîne.

3. Tissu selon la revendication 1, **caractérisé en ce que** lesdits fils sont des fils de trame.

4. Tissu selon l'une ou plusieurs des revendications précédentes, **caractérisé en ce que** lesdits fils sont présents à la fois dans la trame et dans la chaîne.

5. Tissu selon l'une ou plusieurs des revendications précédentes, **caractérisé en ce que** lesdits fils comprenant du cuivre et/ou lesdits fils comprenant du zinc et/ou lesdits fils comprenant du silicium sont alternés avec des fils de trame ou de chaîne faits de fils synthétiques ou naturels.

6. Tissu selon l'une ou plusieurs des revendications précédentes, **caractérisé en ce qu'**il comprend trois groupes de fils: un premier groupe de fils comprenant du cuivre, un deuxième groupe de fils comprenant du zinc, et un troisième groupe de fils comprenant du silicium, lesdits trois groupes de fils étant des fils de chaîne et étant mutuellement alternés en séquence dans la direction de la trame.

7. Tissu selon l'une ou plusieurs des revendications précédentes, **caractérisé en ce qu'**il comprend trois groupes de fils: un premier groupe de fils comprenant du cuivre, un deuxième groupe de fils comprenant du zinc, et un troisième groupe de fils comprenant du silicium, lesdits trois groupes de fils étant des fils de trame et étant mutuellement alternés en séquence dans la direction de la chaîne.

8. Tissu selon la revendication 6, **caractérisé en ce que** lesdits trois groupes de fils sont mutuellement espacés par interposition de fils faits de fils synthétiques ou naturels.

9. Article vestimentaire ou composant d'article vestimentaire **caractérisé en ce qu'**il est réalisé en un tissu selon l'une ou plusieurs des revendications précédentes.

10. Vêtement sanitaire, en particulier du type bandeau élastique, bandage pour cheville, genouillère, bandage pour épaules, gaze, bandeau de poignet, **caractérisé en ce qu'**il est réalisé en un tissu selon l'une ou plusieurs des revendications précédentes.

11. Chaussure ou composant de chaussure, **caractérisé en ce qu'**elle/il est réalisé(e) en un tissu selon l'une ou plusieurs des revendications précédentes.

12. Article de décoration d'intérieur, **caractérisé en ce qu'**il comprend un tissu selon l'une ou plusieurs des revendications précédentes.

13. Revêtement pour composants de décoration d'intérieur, **caractérisé en ce qu'**il comprend un tissu selon l'une ou plusieurs des revendications précédentes.

14. Linge de lit, **caractérisé en ce qu'**il comprend un tissu selon l'une ou plusieurs des revendications précédentes.
